# EUROPEAN PATENT APPLICATION

(11) **EP 4 130 248 A1**
(43) Date of publication of application: **08.02.2023**
(21) Application number: 21775209.6
(22) Date of filing: 23.03.2021
(51) Int. Cl.: C12N 5/0775, A61K 35/28, A61P 13/12, A61P 11/00, A61P 15/08, A61K 8/98, A61Q 7/00, A61Q 19/02

(54) **COMPOSITION FOR CILIOGENESIS PROMOTION, CONTAINING, AS ACTIVE INGREDIENT, MESENCHYMAL STEM CELL OR MESENCHYMAL STEM CELL CULTURE SOLUTION**

(30) Priority: 26.03.2020 KR 20200036871
(71) Applicant: Encell Co., Ltd., Gangnam-gu, Seoul 06072 (KR); Samsung Life Public Welfare Foundation, Seoul 04348 (KR)
(72) Inventor: CHANG, Jong Wook, Seoul 06351 (KR); JEON, Hong Bae, Seoul 05531 (KR); PARK, Sang Eon, Seongnam-si, Gyeonggi-do 13500 (KR)
(74) Representative: dompatent von Kreisler Selting Werner - Partnerschaft von Patent- und Rechtsanwälten mbB
(86) International application number: PCT/KR2021/003583
(87) International publication number: WO 2021/194224

(57) **Abstract**

Provided in the present invention is a composition for ciliogenesis, containing a mesenchymal stem cell or a culture medium of mesenchymal stem cell. The mesenchymal stem cell or the culture medium of mesenchymal stem cell according to the present invention increases the number of primary cilia in cells and elongates same.

The mesenchymal stem cell or the culture medium of mesenchymal stem cell according to the present invention can be used as a pharmaceutical composition for preventing or treating diseases caused by ciliopathy or ciliary impairment.

The mesenchymal stem cell or the culture medium of mesenchymal stem cell according to the present invention can be used as a cosmetic composition.

## Description

### [Technical Field]

This application claims the benefit of priority based on Korean Patent Application No. 10-2020-0036871 filed on March 26, 2020, the entire contents of which are incorporated herein by reference.

The present invention relates to a composition for promoting ciliogenesis, comprising a mesenchymal stem cell or a culture medium of mesenchymal stem cell as an active ingredient.

### [Background Art]

Since stem cells may be differentiated into various cells, various studies are being conducted on the applicability of cell therapy using the characteristics of these stem cells. Embryonic stem cells with pluripotency have attracted attention as cell therapeutic agents due to the ability to differentiate into various cells, but there are difficulties in practical application due to safety and ethical issues.

In order to avoid these safety and ethical issues, many researchers have been interested in adult stem cells as an alternative to the embryonic stem cells. The adult stem cells are cells capable of self-proliferating in an undifferentiated state and multi-differentiating into cells of other tissues, and the earliest known adult stem cells are bone marrow stem cells (BMSCs) derived from bone marrow, but it is known that the adult stem cells exist in various regions of our body, such as the umbilical cord, blood, and fat.

Among these adult stem cells, studies using mesenchymal stem cells capable of differentiating into various tissues with high self-replication ability are being actively conducted.

The mesenchymal stem cells have the ability to regenerate muscle, cartilage, bone tissue, ligaments, bone marrow matrix, and the like through differentiation. In addition, the mesenchymal stem cells induce homeostasis restoration (inhibition of inflammation, angiogenesis, cell attraction, immune regulation, etc.) at lesion sites through the action of various proteins secreted by the mesenchymal stem cells themselves. This is called the paracrine of the mesenchymal stem cells. Due to such paracrine, the mesenchymal stem cells are called 'Drug Store' or 'Drug Factory'.

Cilia are the organelles of rising cells such as hairs on the cell surface, and are present in most cells of animals, including the human body, but have long been known as a vestigial organ which is no important functional. However, it is known that the cilia not only regulate signaling and cell cycles of growth factors crucial for maintaining tissue homeostasis in vivo, such as sonic hedgehog (SHH), platelet-derived growth factor (PDGF), Wnt, and Notch, but also serve as a biosignal input/output hub that senses chemical senses such as smell, taste, and osmotic pressure and physical senses such as sight and hearing and generalizes a signal system. Accordingly, attention has been focused on the cilia.

The specific roles of the cilia are as described in Table 1 below.

**[Table 1]**

| Tissue and cell types | Roles of cilia organelles |
|---|---|
| Common functions | Signaling growth factors such as Hedgehog, PDFG, Wnt, etc. and regulating cell cycle |
| Embryonic Node | Detecting fluid flow and signaling material |
| Kidney, bile duct, pancreas | Detecting fluid flow |
| Inner ear sensory hair cells | Generating and maintaining stereo cilia that sense sound (function of Kino cilia) |
| Photoreceptor cells | Modified rhodopsin is located in the outer segment |
| Auditory nerves | Sense sound |
| Osteoblasts/chondrocytes | Presumed to sense pressure transferred to bone and cartilage |
| Sperm | Involved in sperm migration |
| Epidermal tissue of the bronchial tubes | Discharge of mucus |
| Fallopian tube | Presumed to regulate the movement of the fertilized egg for implantation |

Ciliary disorder, so-called 'ciliopathy', is a collective term for diseases caused by the function or structure of abnormal ciliated cells.

Specifically, the ciliopathy has various forms, including not only genetic diseases such as Bardet-Biedl syndrom (BBS), Joubert syndrome, Meckel-Gruber syndrome, or Leber congenital amaurosis, and Alstrom syndrome, but also polycystic kidney disease (PKD), renal cysts, infertility, respiratory disorder, situs inversus, cancer, and metabolic diseases such as diabetes, obesity, and hypertension. However, therapeutic agents for these diseases have not yet been developed.

As specific symptoms of the ciliopathy, obesity occurs in Badette-Biddle syndrome and Alstrom syndrome, and similarly to human genetic ciliary disorder, obesity and bulimia also occur in mice with ciliopathy, but the reason is known to decrease the action of leptin, an appetite suppressant hormone, in hypothalamic neurons.

In addition, there is a report that no cilia in the skin inhibit the generation of hair or the differentiation of keratin, and it is known that in relation to the skin chromogenesis, primary cilia are activated by sonic hedgehog (SHH)-smoothened-GLI2 signaling to inhibit melanogenesis.

Recently, as various functions of the cilia are known, it is considered to cause the imbalance of systemic metabolic homeostasis due to ciliary disorder in connection with the onset of intractable metabolic diseases such as diabetes, obesity, and hypertension. In addition, functional recovery of cilia is emerging as a target for the treatment of various diseases including metabolic diseases.

### [Prior Art Document]

(Patent Document 1) KR 10-2020-0000415
(Patent Document 2) KR 10-2056172

### [Disclosure]

### [Technical Problem]

An object of the present invention is to provide a composition for promoting ciliogenesis, using a mesenchymal stem cell or a culture medium of mesenchymal stem cell.

Another object of the present invention is to provide a composition for treating or preventing diseases caused by ciliopathy, using a mesenchymal stem cell or a culture medium of mesenchymal stem cell.

Another object of the present invention is to provide a cosmetic composition for inducing functions of promoting hair production, promoting differentiation of keratin, and inhibiting melanogenesis, using a mesenchymal stem cell or a culture medium of mesenchymal stem cell.

### [Technical Solution]

In order to achieve the object, the present invention provides a composition for promoting ciliogenesis comprising a culture medium of mesenchymal stem cell as an active ingredient.

Further, the present invention provides a method for treating or preventing diseases caused by ciliopathy by administering a culture medium of mesenchymal stem cell to a subject.

The present invention provides a composition for ciliogenesis comprising a mesenchymal stem cell.

Further, the present invention provides a method for treating or preventing diseases caused by ciliopathy by administering a mesenchymal stem cell to a subject.

The present invention provides a method for inducing ciliogenesis by co-culturing a mesenchymal stem cell and a ciliated cell.

Further, the present invention provides a method for treating diseases caused by ciliopathy by co-culturing a mesenchymal stem cell and a ciliated cell or a cell with abnormality in ciliogenesis ex vivo to induce ciliogenesis and administering the co-cultured ciliated cell or cell with abnormality in ciliogenesis to a subject.

The present invention provides a cosmetic composition for skin whitening and/or ciliogenesis of skin keratinocytes comprising a culture medium of mesenchymal stem cell as an active ingredient.

Further, the present invention provides a method for inducing skin whitening and/or ciliogenesis of skin keratinocytes by applying or treating a cosmetic composition comprising a culture medium of mesenchymal stem cell to the skin.

### [Advantageous Effects]

According to the present invention, when cells are treated with the composition comprising the mesenchymal stem cell as the active ingredient, it is confirmed that the cilium length is increased, so that the culture medium may be used for the prevention or treatment of ciliopathy.

In addition, when the mesenchymal stem cell of the present invention and the cell having ciliary disorder are co-cultured, the cell with the restored function by elongating the cilia of the cells and/or the mesenchymal stem cell can be used for the treatment of ciliopathy.

According to the present invention, when cells are treated with the composition comprising the mesenchymal stem cell as the active ingredient, it is confirmed that the cilium length is increased, so that the culture medium can be used as the cosmetic composition for inducing ciliogenesis of skin keratinocytes.

### [Description of Drawings]

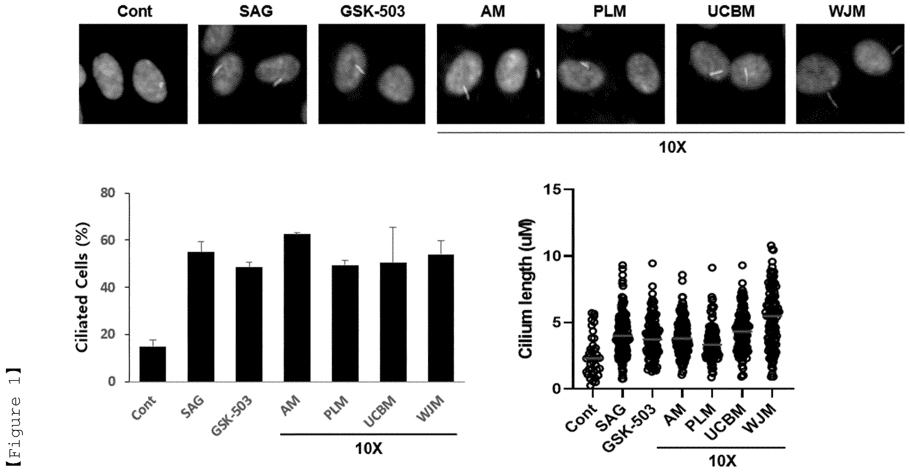

FIG. 1 is a result of primary ciliogenesis when a RPE cell line is treated with a culture medium (10X) of various-derived mesenchymal stem cells isolated and prepared by a method of Example 1. FIG. 1A is a result of observing the shapes of cilia generated in each treatment group through a fluorescence microscope after fluorescence staining (nuclear and cilia double staining), and FIG. 1B is a result of measuring ciliogenesis and a cilium length in each treatment group.

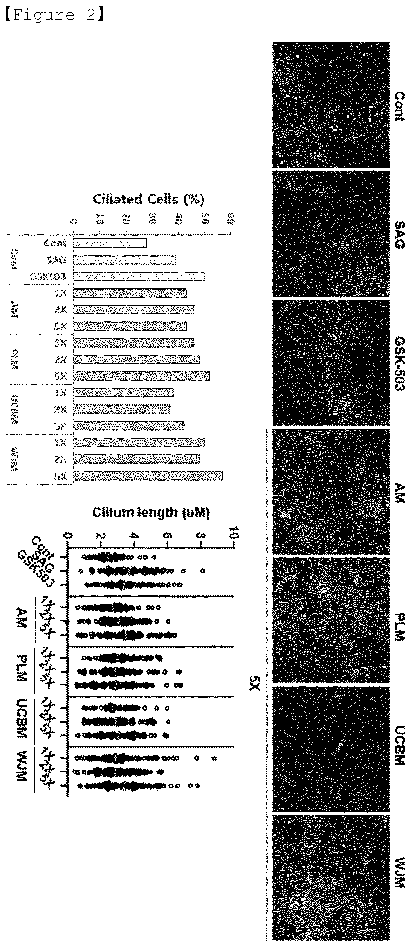

FIG. 2 is a result of primary ciliogenesis when a RPE/Smo-GFP cell line is treated with a culture medium of various-derived mesenchymal stem cells isolated and prepared by a method of Example 1 at various concentrated levels 1X, 2X, and 5X. FIG. 2A is a result of observing the shapes of cells in each treatment group through a fluorescence microscope, and FIG. 2B is a result of measuring ciliogenesis and a cilium length in each treatment group.

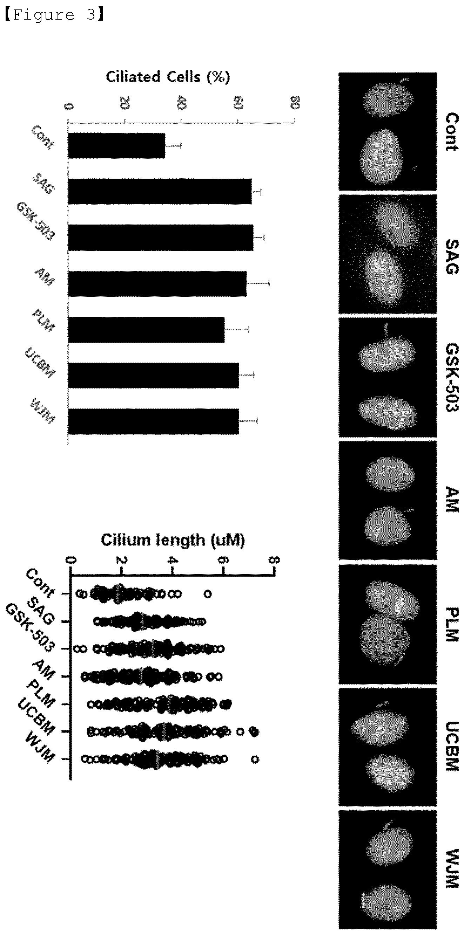

FIG. 3 is a result of primary ciliogenesis in a RPE cell line when the RPE cell line and various-derived mesenchymal stem cells isolated in Example 1 are co-cultured. FIG. 3A is a result of observing the shapes of cilia generated by co-culture with each mesenchymal stem cell through a fluorescence microscope after fluorescence staining (nuclear and cilia double staining), and FIG. 3B is a result of measuring ciliogenesis and a cilium length in each treatment group.

### [Best Mode]

Hereinafter, the present invention will be described in more detail. In the following description, the detailed descriptions of techniques well-known to those skilled in the art may be omitted. Further, in describing the present invention, when it is determined that the detailed description of related known technologies or configurations may unnecessarily limit the gist of the present invention, the description thereof may be omitted. Terminologies used herein are terminologies used to properly express preferred embodiments of the present invention, which may vary according to the intention of a user and an operator, customs in the art to which the present invention pertains, or the like.

Accordingly, definitions of the terminologies need to be described based on contents throughout this specification. Throughout the specification, unless explicitly described to the contrary, when a certain part 'includes' a certain component, it means that other components may be further included, rather than excluding other components.

The present invention provides a composition for ciliogenesis comprising a culture medium of mesenchymal stem cell.

The present invention provides a composition for ciliogenesis comprising a mesenchymal stem cell.

The present invention provides a pharmaceutical composition for preventing or treating ciliopathy or ciliary impairment comprising a culture medium of mesenchymal stem cell.

The present invention provides a pharmaceutical composition for preventing or treating ciliopathy or ciliary impairment comprising a mesenchymal stem cell.

The present invention provides a method of inducing ciliogenesis in abnormal ciliated cells by co-culturing mesenchymal stem cells and abnormal ciliated cells ex vivo.

The present invention provides a cosmetic composition comprising a culture medium of mesenchymal stem cell.

The `ciliopathy' is a collective term for diseases caused by the function or structure of abnormal ciliated cells or cilia. In this specification, the `ciliopathy' and the `disease caused by ciliopathy' are used interchangeably.

The ciliopathy is known to be associated with proteins that are usually localized to motile, non-motile cilia or centrosomes and known to be caused a characteristic caused by a genetic defect, but the related disease genes are not fully known, and a therapeutic agent for the disease has not yet been developed.

The ciliopathy is not particularly limited as long as it is a disease caused by the function or structure of abnormal ciliated cells, and may include, for example, Joubert syndrome, Bardet-Biedl syndrome, Nephronophthisis, Meckel-Gruber syndrome, or Leber congenital amaurosis, polycystic kidney disease (PKD), renal cysts, infertility, respiratory disorders, situs inversus, obesity, hypertension, cancer diseases, and the like.

The ciliopathy or ciliary impairment cells may be selected from cilia epithelial cells, retinal cells, skin cells, and auditory cells, but are not limited thereto.

As used herein, the term 'stem cell' refers to a cell having the ability to differentiate into two or more types of cells while having self-replication ability, and the stem cell is an adult stem cell, a pluripotent stem cell, an induced pluripotent stem, or an embryonic stem cell. The 'adult stem cell' refers to a stem cell that appears in a stage in which each organ of the embryo is formed or in an adult stage as the development process progresses.

As used herein, the term 'mesenchymal stem cell' refers to a mesenchymal-derived pluripotent progenitor cell before differentiation into cells of a specific organ, such as bone, cartilage, fat, tendon, nerve tissue, fibroblasts, and muscle cells. In the present invention, the mesenchymal stem cells are contained in the composition in an undifferentiated state. The mesenchymal stem cells of the present invention may be derived from human umbilical cord, Wharton's jelly, umbilical cord blood, placenta, adipose tissue, bone marrow, tonsils, human embryonic yolk sac, umbilical cord, skin, peripheral blood, muscle, liver, nervous tissue, periosteum, fetal membrane, synovial membrane, synovial fluid, amniotic membrane, meniscus, anterior cruciate ligament, articular chondrocytes, milk teeth, perivascular cells, trabecular bone, subpatellar fat mass, spleen, thymus, etc. Preferably, the mesenchymal stem cells may be derived from at least one selected from the group consisting of human umbilical cord, Wharton's jelly, umbilical cord blood, placenta, fat, bone marrow and tonsils, and preferably, derived from human umbilical cord, Wharton's jelly, umbilical cord blood, or adipose tissue, but the origin thereof is not limited.

In addition, the mesenchymal stem cells are preferably derived from humans, but may also be derived from fetuses or other mammals other than humans. The mammals other than humans may be dogs, cats, monkeys, cow, sheep, pig, horse, rat, mouse, guinea pig, or the like, and the origin thereof is not limited.

In the present invention, the term `adipose tissue-derived mesenchymal stem cells (AMs)' may be isolated from human adipose tissue. Suitable human adipose tissue may refer to including mature adipocytes and connective tissue surrounding the adipocytes. The human adipose tissue may use all adipose tissues obtained by any method used for collecting fat regardless of the body location of an individual.

In the present invention, `placenta-derived mesenchymal stem cells (PLMs)' include all stem cells isolated from the placenta, and preferably, may include four types of stem cells isolated from the human placenta isolated ex vivo, that is, (1) human amniotic epithelial cells (hAECs), (2) human amniotic mesenchymal stromal cells or human amniotic mesenchymal stem cells (hAMSCs), (3) human chorionic mesenchymal stromal cells or human chorionic mesenchymal stem cells (hCMSCs), and (4) human chorionic trophoblastic cells (hCTCs).

In the present invention, `umbilical cord blood derived mesenchymal stem cells (UCBMs)' refer to only mesenchymal stem cells excluding blood stem cells among stem cells present in the umbilical cord after delivery.

In addition, 'wharton's Jelly-derived mesenchymal stem cells (WJM)' include all stem cells isolated from the wharton's Jelly, and may mean a string that connects the mother and the embryo so that the fetus in mammals can grow in the placenta, and may generally refer to a tissue consisting of three blood vessels, that is, two umbilical arteries and one umbilical vein, surrounded by Wharton's Jelly.

The active ingredient of the composition for ciliogenesis of the present invention is a mesenchymal stem cell, a culture medium of mesenchymal stem cell or a concentrate thereof, and an extract of the active ingredient, but is not limited.

As used herein, the term 'culture media' refers to a culture medium that can support stem cell growth and survival under ex-vivo culture conditions, and includes all conventional media used in the field suitable for culturing the stem cells. In addition, the culture medium and culture conditions may be selected according to a type of cell. The culture medium used for culture includes a Dulbecco's modified Eagle's medium (DMEM), a minimal essential medium (MEM), a basal medium Eagle (BME), RPMI1640, F-10, F-12, α-modified minimum essential media (α-MEM), a Glasgow's minimal essential medium (GMEM), an Iscove's modified Dulbecco's medium (IMDM), etc., but is not necessarily limited thereto.

In the present invention, the culture medium is based on α-modified minimum essential media (α-MEM) used for cell culture in the industry, and includes serum commonly used for cell culture.

It is preferred to use 0.1 to 20% fetal bovine serum (FBS) as the serum, and it is preferred to use antibiotics, antifungals, and materials commonly used in the industry to prevent the growth of mycoplasma causing contamination.

The antibiotics can use all antibiotics commonly used for cell culture, such as penicillin-streptomycin, the antifungals can use alporericin B, and the mycoplasma inhibitors preferably use materials generally used, such as gentamicin, ciproflolsacin, azithromycin, etc.

In the present invention, after mesenchymal stem cells are isolated from various mesenchymal tissues such as human umbilical cord, placenta, umbilical cord blood, and adipose tissue by a known method suitable for each tissue, each mesenchymal stem cell is subcultured by adding a-modified minimum essential media (α-MEM) containing 10% fetal bovine serum (FBS) and 0.5% gentamicin (10 mg/ml).

The culture medium of mesenchymal stem cell for ciliogenesis is concentrated (2X, 5X, and 10X), or used at an original concentration (1X) as a culture medium obtained after culturing 4-subcultured mesenchymal stem cells (P4) in the α-MEM containing 10% fetal bovine serum (FBS) and 0.5% gentamicin (10 mg/ml) and culturing the mesenchymal stem cells only in a serum-free α-MEM except for FBS for 24 hours when 70% confluency is reached.

The various-derived culture medium of mesenchymal stem cell (5X and 10X) of the present invention showed a result of inducing primary ciliogenesis at the same level as or higher level than SAG and GSK-503, which are positive controls, in retinal pigment epithelial cells (RPE cells).

In the present invention, a co-culture method with mesenchymal stem cells may be used for the primary ciliogenesis of cells. The mesenchymal stem cell of the present invention and a target cell to induce ciliogenesis are co-cultured using a commonly used Transwell^{™} chamber system. Specifically, by culturing the target cell to induce ciliogenesis in the lower part of a well and the mesenchymal stem cell in a Transwell based on a permeable membrane, while blocking direct contact between the two types of cells, the target cell receives a factor secreted from the mesenchymal stem cell to generate cilia.

Even by co-culture of various-derived mesenchymal stem cells of the present invention and retinal pigment epithelial cells (RPE cells), a result of inducing primary ciliogenesis was shown at the same level as or higher level than SAG and GSK-503, which are positive controls.

The present invention provides a method for treating or preventing ciliopathy, which is a disease caused by ciliary disorder, by administering a culture medium of mesenchymal stem cell or a mesenchymal stem cell to a subject.

The present invention provides a cell therapeutic agent for treating ciliopathy by isolating cells having ciliary disorder from a patient, treating the cells with a culture medium of mesenchymal stem cell or *Ex-vivo* co-culturing the cells with a mesenchymal stem cell to improve ciliogenesis, and then injecting the cells to the patient again.

In addition, the present invention provides a method for treating ciliopathy including isolating cells having ciliary disorder from a patient, treating the cells with a culture medium of mesenchymal stem cell or *Ex-vivo* co-culturing the cells with a mesenchymal stem cell to improve ciliogenesis, and then injecting the cells to the patient again.

The present invention provides a cosmetic composition for inducing functions of promoting hair generation, promoting differentiation of keratin, and inhibiting melanogenesis through ciliogenesis promotion using a culture medium of mesenchymal stem cell.

In addition, the present invention provides a method for inducing skin whitening and/or ciliogenesis of skin keratinocytes by applying or treating a cosmetic composition containing a culture medium of mesenchymal stem cell to the skin.

When the composition of the present invention is prepared as a pharmaceutical composition, the pharmaceutical composition of the present invention includes a pharmaceutically acceptable carrier. The pharmaceutically acceptable carrier to be included in the pharmaceutical composition of the present invention includes ingredients selected from lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate and mineral oil, but is not limited thereto.

The pharmaceutical composition of the present invention may further include a lubricant, a wetting agent, a binder, a lubricant, a surfactant, a filler, a disintegrant, a thickener, a stabilizer, a diluent, a preservative, a sweetener, and the like, in addition to the carrier ingredients. Suitable pharmaceutically acceptable carriers and other agents are within the level of those of ordinary skill in the art.

The pharmaceutical composition according to the present invention may be formulated and used in the form of oral formulations such as powders, granules, tablets, capsules, suspensions, emulsions, syrups, aerosols, etc., external preparations, suppositories, and sterile injectable solutions according to a conventional method.

The pharmaceutical composition of the present invention may be administered orally or parenterally, and in the case of parenteral administration, the pharmaceutical composition may be administered intravenously, subcutaneously, intramuscularly, intraperitoneally, or transdermally.

A suitable dosage of the pharmaceutical composition of the present invention is determined within the level of those of ordinary skill in the art, and may vary depending on a formulation method, an administration method, and age, weight, stage, health condition, and the like of a patient. An effective dosage may be estimated from a dose-response curve obtained from ex-vivo experiments or animal model tests.

The pharmaceutical composition of the present invention may be for treating diseases associated with cilia expression abnormality. The diseases associated with the cilia expression abnormality have various forms including polycystic kidney disease (PKD), renal cysts, infertility, respiratory disorder, situs inversus, cancer, and metabolic diseases such as diabetes, obesity, and hypertension, as well as human genetic ciliopathy, Bardet-Biedl syndrom (BBS) or Alstrom syndrome.

When the composition of the present invention is prepared as the cosmetic composition, the composition may be prepared even in any formulation commonly prepared in the art, and may be formulated by, for example, a solution, a suspension, an emulsion, a paste, a gel, a cream, a lotion, a powder, soap, a surfactant-containing cleansing, oil, a powder foundation, an emulsion foundation, a wax foundation, a spray, and the like, but is not limited thereto. More specifically, the composition of the present invention may be prepared in formulations of softener, nourishing toner, nourishing cream, massage cream, essence, eye cream, cleansing cream, cleansing foam, cleansing water, pack, spray or powder.

When the formulation of the present invention is the paste, cream, or gel, as the carrier ingredient, animal oils, vegetable oils, wax, paraffin, starch, cellulose derivatives, polyethylene glycol, silicone, bentonite, silica, talc, zinc oxide or the like may be used.

When the formulation of the present invention is the powder or spray, as the carrier ingredient, lactose, talc, silica, aluminum hydroxide, or calcium silicate may be used, and particularly, in the case of the spray, a propellant such as chlorofluorohydrocarbon, propane/butane or dimethyl ether may be additionally included.

When the formulation of the present invention is the solution or emulsion, as the carrier ingredient, a solvent, a solubilizing agent or an emulsifying agent may be used. For example, the carrier ingredient includes water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the formulation of the present invention is the suspension, as the carrier ingredient, a liquid diluent such as water, ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used.

When the formulation of the present invention is the surfactant-containing cleansing, as the carrier ingredient, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, aliphatic alcohol, fatty acid glyceride, fatty acid diethanolamide, vegetable oil, lanolin derivatives, ethoxylated glycerol fatty acid ester, or the like may be used.

The ingredients included in the cosmetic composition of the present invention include ingredients commonly used in the cosmetic compositions, and for example, conventional adjuvants, such as antioxidants, stabilizers, solubilizers, vitamins, pigments and fragrances, in addition to the culture medium of mesenchymal stem cell as the active ingredient and the carrier ingredient.

The content of the mesenchymal stem cell or the culture medium thereof contained in the cosmetic composition of the present invention is not limited thereto, but is more preferably 0.001 to 10 wt%, 0.01 to 5 wt% based on the total weight of the total composition. When the content thereof is less than 0.001 wt%, it cannot be expected to promote desired hair generation, whitening, or differentiation of skin keratinocytes, and when the content thereof is more than 10 wt%, there may be difficulties in safety or manufacture of formulations.

Hereinafter, preferred Examples will be provided to help in understanding of the present invention. However, the following Examples are just illustrative of the present invention, and it will be apparent to those skilled in the art that various changes and modifications can be made within the scope and the technical idea of the present invention and it is natural that these changes and modifications are within the scope of the appended claims.

### Example 1: Preparation of culture medium of umbilical cord tissue-derived mesenchymal stem cells

Human umbilical cord, placenta, and umbilical cord blood were collected with the consent of mothers after the approval of the Institutional Review Board (IRB) of Samsung Medical Center, and adipose tissue was also collected with the consent of patients after IRB approval of Samsung Medical Center.

Human placenta-derived mesenchymal stem cells (PLM) were isolated from the isolated placenta according to a previously reported method of CHOI and the like (PLoS ONE, (2017) 12 (2) : e0172642), umbilical cord blood-derived mesenchymal stem cells (UCBM) were isolated from the isolated umbilical cord blood according to a method of KIM and the like (FEBS Letters, (2010) 584: 3601-3608), and wharton's Jelly-derived mesenchymal stem cells (WJM) were isolated from the isolated umbilical cord according to a method of PARK and the like (Arch. Pharm. Res. (2016) 39: 1171-1179).

Adipose tissue-derived mesenchymal stem cells (AM) were isolated from the isolated adipose tissue according to a previously reported method of PALUMBO and the like (Int. J. Mol. Sci. (2018) 19: 1987).

Each of the obtained mesenchymal stem cells was subcultured by adding α-modified minimum essential media (α-MEM) containing 10% fetal bovine serum (FBS) and 0.5% gentamicin (10 mg/ml).

A culture medium of mesenchymal stem cell for primary ciliogenesis was concentrated or used at an original concentration as a culture medium obtained after culturing 4-subcultured mesenchymal stem cells (P4) in the α-MEM containing 10% fetal bovine serum (FBS) and 0.5% gentamicin (10 mg/ml) and culturing the mesenchymal stem cells only in a serum-free α-MEM except for FBS for 24 hours when 70% confluency was reached.

The culture medium of mesenchymal stem cell was concentrated 2X, 5X, or 10X using an Amicon Ultra 3K filter unit (MERCK).

### Example 2: Ciliogenesis in RPE cells during treatment with culture medium of mesenchymal stem cell (10X)

In order to analyze a primary cilia growth effect of a culture medium of mesenchymal stem cell, human telomeraseimmotalized retinal pigment epithelium cells (hTERT RPE-1 cell line, CRL-4000; RPE cell line) were purchased from ATCC and used.

The RPE cell line was cultured in α-MEM added with 10% (v/v) fetal bovine serum (FBS) and 0.5% gentamicin at 37°C in a 5% (v/v) CO₂ incubator.

The cultured RPE cell line was inoculated into a 12-well plate (8 × 10⁴ cells/well), cultured for 24 hours, and then stabilized, and the medium was changed to a 10-fold concentrated culture medium of mesenchymal stem cell prepared in Example 1, and the RPE cells were observed after 24 hours to analyze the generated primary cilia. In the RPE cell line, 1 mM SAG and 10 mM GSK-503 were added to the α-MEM as a positive control material for ciliogenesis, and only the α-MEM was used as a negative control for culturing.

When the primary cilia generated from the RPE cell line were cultured, the primary cilia were fluorescently stained with ARL13B and the nuclei were fluorescently stained with Hoechst33342, and then, the numbers of cells and primary cilia were directly counted under a fluorescence microscope, and the lengths of primary cilia were directly measured, and then the number and the length of the primary cilia were analyzed.

After staining, the results observed with the fluorescence microscope were shown in FIG. 1A, and the results of analyzing the ratio of cells in which primary cilia have occurred and the length of the generated cilia were shown in FIG. 1B.

From the results of FIG. 1B, when culture medium of mesenchymal stem cell isolated from adipose tissue (AM), placenta (PLM), umbilical cord blood (UCBM), and Wharton's jelly (WJM) tissues were 10-fold concentrated and treated under conditions of inducing ciliogenesis in the RPE cell line, it can be confirmed that primary cilia were generated at the same level as or higher level than positive controls SAG and GSK-503 (3 to 5 times higher than the negative control, and about 1.2 times higher than the positive control).

In addition, under the same conditions, it can be confirmed that the cilium length is also longer than that of the negative control and the positive control (1.5 to 2 times higher than the negative control, and about 1.2 times higher than the positive control) .

### Example 3: Ciliogenesis in RPE cells during treatment with culture medium of mesenchymal stem cell according to concentration (1X, 2X, and 5X)

In order to directly analyze the actually generated cilia, an RPE/Smo-GFP cell line expressing a smoothened protein (Smo)-GFP fusion gene specifically expressed in cilia was used as the RPE cell line.

1X, 2X, and 5X concentrates of each mesenchymal stem cell (adipose (AM), placenta (PLM), umbilical cord blood (UCBM), and Wharton's jelly (WJM)) culture medium were treated in the RPE/Smo-GFP cell line under the same conditions for inducing ciliogenesis as Example 1 and then the ciliogenesis and the length thereof were analyzed using a fluorescence microscope.

After culturing, the results observed with the fluorescence microscope were shown in FIG. 2A, and the results of analyzing the ratio of cells in which primary cilia have occurred and the length of the generated cilia were shown in FIG. 2B.

From the result of FIG. 2B, a phenomenon of promoting the ciliogenesis and increasing the cilium length in proportion to the concentration of the culture medium of mesenchymal stem cell could be confirmed. Particularly, it can be seen that the ciliogenesis of the RPE/Smo-GFP cell line was promoted at the same as or higher level than the positive control at a concentration of 5X of the culture medium (1.5 to 2 times higher than the negative control, and about 1.2 times higher than the positive control). In addition, it was confirmed that the cilium length was also increased at the concentration of 5X of the culture medium (1.5 to 2 times higher than the negative control).

### Example 4: Ciliogenesis in RPE cells during co-culturing with mesenchymal stem cells

Co-culturing of a RPE cell line and mesenchymal stem cells was performed using a Transwell^{™} chamber in a serum-free state, the RPE cell line was cultured in the lower part of the well, and the mesenchymal stem cells were cultured in the Transwell. At this time, in the positive control, the RPE cells were cultured in the lower part and the Transwell was cultured by adding 1 mM SAG and 10 mM GSK-503 to the α-MEM, and in the negative control, the RPE cell line was cultured in the lower part, and the Transwell was cultured and used by adding the α-MEM.

At this time, the same number of RPE cell lines were cultured in the lower parts of the negative control, the positive control, and the mesenchymal stem cell co-culture group, and cultured in the same amount of culture medium.

After the co-culturing was completed, the RPE cells in the lower well were fluorescently stained in the same manner as in Example 2 to analyze the ciliogenesis and length.

After culturing, the results observed with the fluorescence microscope were shown in FIG. 3A, and the results of analyzing the ratio of cells in which primary cilia have occurred and the length of the generated cilia were shown in FIG. 3B.

It was confirmed that the co-culturing with the mesenchymal stem cells promoted the ciliogenesis of the RPE cell line at the same level as or higher level than the positive controls SAG and GSK-503 (2 to 2.5 times higher than the negative control, and about 1.1 times higher than the positive control), and the cilium length was also increased (1.5 to 2 times higher than the negative control, and 1.2 to 1.3 times higher than the positive control).

Although the present invention has been described with reference to the limited examples and drawings, it is to be understood by the person skilled in the art that the present invention is not limited thereto and that various modifications and variations are possible within the technical idea of the present invention and the scope equivalent to the claims set forth below.

## Claims

1. A composition for ciliogenesis comprising a culture medium of mesenchymal stem cell.

2. The composition for ciliogenesis according to claim 1, wherein the mesenchymal stem cell is a mesenchymal stem cell derived from bone marrow matrix, adipose tissue, umbilical cord, cartilage, placenta, or umbilical cord blood.

3. A composition for ciliogenesis comprising a mesenchymal stem cell.

4. The composition for ciliogenesis according to claim 3, wherein the mesenchymal stem cell is a mesenchymal stem cell derived from bone marrow matrix, adipose tissue, umbilical cord, cartilage, placenta, or umbilical cord blood.

5. A pharmaceutical composition for preventing or treating ciliopathy or ciliary impairment comprising a culture medium of mesenchymal stem cell.

6. The pharmaceutical composition according to claim 5, wherein the mesenchymal stem cell is a mesenchymal stem cell derived from bone marrow matrix, adipose tissue, umbilical cord, cartilage, placenta, or umbilical cord blood.

7. The pharmaceutical composition according to claim 5 or 6, wherein the ciliopathy is selected from Bardet-Biedl syndrom (BBS), Alstrom syndrome, polycystic kidney disease (PKD), renal cysts, respiratory disorder, infertility, situs inversus, cancer, and metabolic diseases such as diabetes, obesity, and hypertension.

8. A pharmaceutical composition for preventing or treating ciliopathy or ciliary impairment comprising a mesenchymal stem cell.

9. The pharmaceutical composition according to claim 8, wherein the mesenchymal stem cell is a mesenchymal stem cell derived from bone marrow matrix, adipose tissue, umbilical cord, cartilage, placenta, or umbilical cord blood.

10. The pharmaceutical composition according to claim 8 or 9, wherein the ciliopathy is selected from Bardet-Biedl syndrom (BBS), Alstrom syndrome, polycystic kidney disease (PKD), renal cysts, respiratory disorder, infertility, situs inversus, obesity, diabetes, hypertension, and cancer diseases.

11. A method for inducing ciliogenesis in an abnormal ciliated cell by co-culturing a mesenchymal stem cell and the abnormal ciliated cell ex vivo.

12. The method of claim 11, wherein the mesenchymal stem cell is a mesenchymal stem cell derived from bone marrow matrix, adipose tissue, umbilical cord, cartilage, placenta, or umbilical cord blood.

13. The method according to claim 11 or 12, wherein the abnormal ciliated cell is a cell with abnormality in ciliogenesis selected from cilia epithelial cells, retinal cells, skin cells, and auditory cells.

14. A cosmetic composition for ciliogenesis comprising a culture medium of mesenchymal stem cell.

15. The cosmetic composition according to claim 14, wherein the mesenchymal stem cell is a mesenchymal stem cell derived from bone marrow matrix, adipose tissue, umbilical cord, cartilage, placenta, or umbilical cord blood.

16. The cosmetic composition according to claim 14 or 15, wherein the cosmetic composition is for promoting hair generation, skin whitening, or protecting cells due to skin keratogenesis.
